# EUROPÄISCHE PATENTSCHRIFT

(11) **EP 1 531 881 B1**
(45) Veröffentlichungstag und Bekanntmachung des Hinweises auf die Patenterteilung: **16.06.2010**
(21) Anmeldenummer: 03762454.1
(22) Anmeldetag: 07.07.2003
(51) Int. Cl.: A61M 1/00

(54) **CHIRURGISCHE EINRICHTUNG ZUR ENTNAHME VON GEWEBEZELLEN AUS EINER BIOLOGISCHEN STRUKTUR INSBESONDERE ZUR LIPOSUKTION**
SURGICAL DEVICE FOR REMOVING TISSUE CELLS FROM A BIOLOGICAL STRUCTURE, ESPECIALLY FOR LIPOSUCTION
INSTRUMENT CHIRURGICAL PERMETTANT DE PRELEVER DES CELLULES TISSULAIRES ISSUES D'UNE STRUCTURE BIOLOGIQUE EN PARTICULIER POUR LA LIPOSUCTION

(30) Priorität: 09.07.2002 DE 20210646 U
(43) Veröffentlichungstag der Anmeldung: 25.05.2005
(73) Patentinhaber: Human Med AG, 19061 Schwerin (DE)
(72) Erfinder: PEIN, Andreas, 23911 Einhaus (DE)
(74) Vertreter: Schneider, Henry
(86) Internationale Anmeldenummer: PCT/DE2003/002256
(87) Internationale Veröffentlichungsnummer: WO 2004/004788

(56) Entgegenhaltungen:
- WO-A-03/039629
- DE-A- 10 033 278
- US-A- 5 242 387
- US-A- 5 499 970
- US-A- 5 573 504
- US-A- 5 759 178
- US-A- 5 836 909

## Beschreibung

Die Erfindung bezieht sich auf eine Einrichtung nach dem Oberbegriff des Anspruchs 1. Derartige Einrichtungen und Instrumente werden in chirurgischen Kliniken eingesetzt, um aus gesundheitlichen oder kosmetischen Gründen Fettgewebe abzusaugen. Solche Einrichtungen und Instrumente werden aber auch zur Entnahme von vitalen Gewebezellen zum Beispiel aus einer Leber verwendet, um diese Gewebezellen auf dem Wege der Teilung zu vermehren und sie dann an der gleichen oder an einer anderen biologischen Struktur wieder einzusetzen.

Dazu sind eine Reihe von Verfahren und Einrichtungen bekannt.
So ist zum Beispiel aus der DE 299 14 230 U1 eine Kanüle für die Fettabsaugung bekannt, die aus einem Rohr gebildet ist, das einerseits verschlossen und andererseits über einen Adapter mit einer Sauganlage verbunden ist. Das Rohr ist mit mehreren am Umfang verteilt angeordneten Saugöffnungen ausgestattet, die in ihrer Größe auf die Größe der Fettgewebezellen abgestimmt sind.
Diese Kanüle wird in die entsprechenden Gewebeschichten eingestochen und während des Eingriffs ständig hin- und herbewegt. Unter der Kraft des Vakuums und mit der Unterstützung der mechanischen Kraft der bewegten Kanüle werden Gewebezellen in zerstörender Weise abgerissen und dann abgesaugt. Dieses Verfahren ist für den Patienten sehr belastend und wird daher in der Praxis kaum noch angewendet.

Zur Verringerung dieser Belastung ist es allgemein bekannt, in einem separaten Arbeitsgang vor dem chirurgischen Eingriff eine Arbeitsflüssigkeit in das betreffende Gewebe einzuspritzen, damit sich dadurch die Gewebezellen lösen und so besser und leichter abgesaugt werden können. Dabei wird die eingespritzte Arbeitsflüssigkeit wieder zusammen mit den gelösten Gewebezellen über das Saugrohr abgesaugt.

In der DE 200 09 786 U1 wird nun eine Fettabsaugvorrichtung vorgestellt, die diese beiden Arbeitsgänge des Einspritzens einer gewebelösenden Arbeitsflüssigkeit und des Absaugens der gelösten Gewebezellen funktionell miteinander verbindet. Dazu ist in der Absaugkanüle eine innenliegende Einspritzleitung für die gewebelösende Arbeitsflüssigkeit angeordnet, deren Austrittsöffnung sich am distalen Ende der Absaugkanüle befindet und die andererseits mit einer Arbeitsflüssigkeitspumpe verbunden ist. Auf diese Weise wird kontinuierlich Arbeitsflüssigkeit eingespritzt und gleichzeitig und zusammen mit den Fettgewebezellen wieder abgesaugt. Damit wird der Eingriff verkürzt und der Ablauf kontinuierlicher.

Alle bisher genannten technischen Lösungen haben aber gemeinsam den wesentlichen Nachteil, dass nicht nur die Fettgewebezellen sondern auch benachbarte Gewebezellen, wie beispielsweise Blutgewebezellen zerstört werden. Das beschädigt den menschlichen Körper und kompliziert und verlängert den anschließenden Heilungsprozess. Diese technischen Lösungen sind daher auch nicht geeignet, gesunde Gewebezellen zum Zwecke der Weiterverwendung zu entnehmen.

Diesen Nachteil beseitigt eine chirurgische Einrichtung zur Entnahme von Gewebezellen aus einer biologischen Struktur, die in DE 100 33 278 A1 vorgestellt wird. Diese Einrichtung besteht aus einer Wasserstrahleinrichtung mit einem Druckerzeuger und einer Einspritzkanüle, aus der ein trennender Wasserstrahl unter Druck austritt und aus einer Saugeinrichtung mit einer Saugpumpe und einem Saugrohr mit am Umfang verteilten Saugöffnungen, durch das die abgetrennten Gewebezellen mit dem verbrauchten Wasser abgeleitet werden. Dabei ist die Einspritzkanüle für den austretenden Wasserstrahl im Inneren des Saugrohres angeordnet und beide sind in einem Handstück vereint, das über einen schraubbaren Adapter auswechselbar ausgeführt ist.
Dabei besitzt die Austrittsöffnung der Einspritzkanüle einen solchen Querschnitt und der austretende Wasserstrahl einen solchen Druck, dass der austretende Wasserstrahl eine schälende Wirkung bekommt.
Dieser Wasserstrahl ist in der Lage, Gewebeteile zu zerschneiden oder zu zertrennen. Bemerkenswerterweise werden aber nicht die Gewebezellen zerstört, da der Wasserstrahl wegen der gewölbten Oberfläche und der Nachgiebigkeit der Gewebezellen keinen Widerstand erfährt und dadurch in seiner Wirkungsrichtung abgelenkt wird. So findet der Wasserstrahl in intelligenter Weise seinen Weg zwischen die Gewebezellen. Erst hier findet er den Widerstand, den er zur Entfaltung seiner Trennkraft benötigt und so drängt er benachbarte Gewebezellen auseinander und trennt sie voneinander, ohne sie zu zerstören. Wegen dieser schonenden Selektierung wird das vorherige Einspritzen einer Arbeitsflüssigkeit zum Zwecke des Lösens der Gewebezellen überflüssig.
Die relativ hohe Zahl der Anwendungsfälle und die hohe Anzahl der durchmesserbedingten Ausführungsformen eines solchen Handstückes einerseits und die Forderung nach einer hohen Auslastungsquote für die gesamte Einrichtung andererseits verlangen danach, eine Vielzahl von Handstücken bereitzuhalten, die sich in der Länge, im Durchmesser des Saugrohres und im Durchmesser der Absaugöffnungen des Saugrohres unterscheiden. Diese Werkzeugvielfalt verteuert aber die gesamte Einrichtung in unvertretbarer Weise.

Außerdem macht es sich in vielen Fällen erforderlich, vor dem chirurgischen Eingriff ein Anästhetikum zu verabreichen, wozu ein spezielles Injektionsbesteck verwendet wird. Das erhöht weiterhin den vorrichtungsseitigen Aufwand eines solchen chirurgischen Eingriffs und auch die erforderliche Zeit zur Umrüstung von der Injektionsvorrichtung zur Gewebeentnahmeeinrichtung und umgekehrt.

Der Erfindung liegt daher die Aufgabe zu Grunde, eine gattungsgemäße Einrichtung zur Entnahme von Gewebezellen zu entwickeln, die universell für die Anästhesie und für die Gewebeentnahme eingesetzt werden kann und die dabei einfach im Aufbau und in der Handhabung ist.

Diese Aufgabe wird durch die kennzeichnenden Merkmale des Anspruches 1 gelöst. Weitere Ausgestaltungsmöglichkeiten ergeben sich aus den Unteransprüchen 2 bis 5. Die neue chirurgische Einrichtung beseitigt die genannten Nachteile des Standes der Technik.
Der besondere Vorteil der neuen chirurgischen Einrichtung liegt in der universellen Einsatzfähigkeit. So kann die aus dem Handgriff und der Einspritzkanüle bestehende Grundausstattung mit den verschiedensten Komplettierungsteilen bestückt werden und so an den jeweils speziellen Einsatzfall angepasst werden. Dabei ist der Wechselvorgang der Komplettierungsteile sehr einfach und schnell durchführbar, wodurch wertvolle Operationszeit gespart wird.
Erhebliche Vorrichtungs- und Einrichtungskosten werden dadurch gespart, dass nur noch eine Versorgungseinrichtung erforderlich wird und für die unterschiedlichsten Anwendungsfälle nicht mehr komplette Operationshandstücke, sondern nur noch einzelne Komplettierungsteile ausgetauscht werden müssen. Auch das spart Operationszeit.

Die Erfindung soll anhand eines Ausführungsbeispieles näher erläutert werden.
Dazu zeigen:
- Fig. 1:: die neue chirurgische Einrichtung in einer vereinfachten schematischen Darstellung,
- Fig. 2:: das Operationshandstück in seiner Grundausführung mit der Einzelheit X,
- Fig. 3:: ein erstes Komplettierungsteil in Form eines Stabilisierungsgriffs für die Einspritzkanüle,
- Fig. 4:: ein zweites Komplettierungsteil in Form eines Stabilisierungsgriffs mit einem Stabilisierungsrohr und
- Fig. 5:: ein drittes Komplettierungsteil in Form eines Stabilisierungsgriffs mit einem Absaugrohr und den Einzelheiten X und A-A.

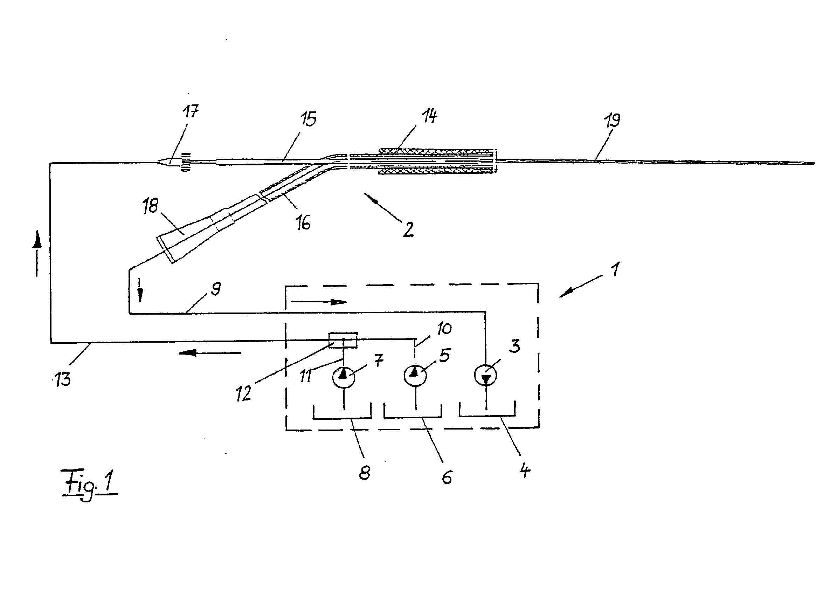

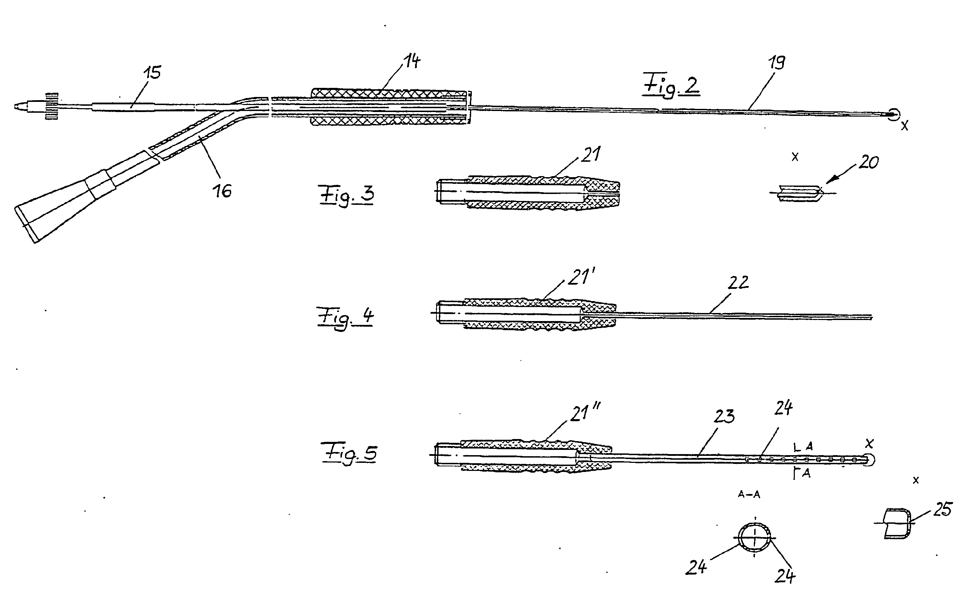

Nach der Fig. 1 besteht die chirurgische Einrichtung aus einer Versorgungseinrichtung 1 und einem Operationshandstück 2. Zur Versorgungseinrichtung 1 gehören eine Vakuumpumpe 3 mit einem Aufnahmebehälter 4, eine Versorgungspumpe 5 mit einem Versorgungsbehälter 6 für eine sterile und strahlfähige Trennflüssigkeit und eine Versorgungspumpe 7 mit einem Versorgungsbehälter 8 für ein Anästhetikum oder einer anderen Arbeitsflüssigkeit.
Die Vakuumpumpe 3 ist über eine Unterdruckleitung 9 mit dem Operationshandstück 1 verbunden. Dagegen besitzen die beiden Versorgungspumpen 5 und 7 jeweils eine Druckleitung 10 und 11, die beide in ein schaltbares Wegeventil 12 münden. Dieses Wegeventil 12 ist für den alternativen Einsatz einer der beiden Versorgungspumpen 5 und 7 vorgesehen und ist verbraucherseitig über eine Druckversorgungsleitung 13 mit dem Operationshandstück 1 verbunden.
Das Operationshandstück 1 besteht, wie es auch die Fig. 2 näher zeigt, aus einem Handstück 14 mit einer innenliegenden Einspritzleitung 15 und einer, diese Einspritzleitung 15 umgebende Absaugleitung 16. Auf der proximalen Seite des Handstückes 14 ist die Einspritzleitung 15 aus der Absaugleitung 16 herausgeführt und über ein Kupplungsstück 17 mit der Druckversorgungsleitung 13 verbunden. Dagegen besitzt die Absaugleitung 16 ein Kupplungsstück 18, das Verbindung zur Unterdruckleitung 9 besitzt. Auf der distalen Seite des Handstückes 14 befindet sich eine feststehende und mit der Einspritzleitung 15 verbundene Einspritzkanüle 19. Diese Einspritzkanüle 19 ist an ihrem freien Ende als eine kegelspitzartig auslaufende Einspritzdüse ausgebildet und besitzt vorzugsweise im Bereich der Kegelmantelfläche eine als Schlitz ausgebildete Düsenöffnung 20. Wegen der Anordnung der Düsenöffnung 20 in der Kegelmantelfläche tritt der Flüssigkeitsstrahl in einer von der Achse der Einspritzkanüle 19 abweichenden Richtung aus. Dabei ist diese Düsenöffnung 20 so bemessen, dass in Verbindung mit einem ausgewählten Druck in der Druckversorgungsleitung 13 ein überwiegend flacher Flüssigkeitsstrahl austritt, der eine schälende Trennwirkung gegenüber den Gewebezellen ausübt.
Die Einspritzkanüle 19 ist in ihrer Länge so bemessen, dass auch tieferliegende Gewebebereiche erreichbar sind. Da die relativ lange und dünne Einspritzkanüle 19 eine gewisse Instabilität aufweist und andererseits für die Einspritzung eines Ästhetikums nur eine relativ geringe Einstichtiefe nötig ist, ist ein erstes Komplettierungsteil zur Stabilisierung der Einspritzkanüle 19 für die Injektion eines Ästhetikums vorgesehen, wie es die Fig. 3 zeigt. Dieses Komplettierungsteil besteht aus einem Stabilisierungshandgriff 21 und wird auf die Einspritzkanüle 19 aufgeschoben und mit dem Handstück 14 verschraubt. Dadurch verlängert sich die Führungs- und Auflagelänge am Handstück 14. Aus ergonomischen Gründen ist der Stabilisierungshandgriff 21 und das Handstück 14 mit gleichen äußeren Abmessungen und einer angepassten Gestaltung versehen.
Die Fig. 4 zeigt ein weiteres Komplettierungsteil, das aus einem Stabilisierungshandgriff 21' und einem daran befestigten Stabilisierungsrohr 22 besteht, welches die Einspritzkanüle 19 über die gesamte Länge abstützt. Dabei ist die Einspritzkanüle 19 an ihrem freien Ende offen und besitzt eine solche Länge, dass die Düsenöffnung 20 der Einspritzkanüle 19 mit einem ausreichenden Abstand aus dem Stabilisierungsrohr 22 herausragt. Mit dieser stabilisierten Einspritzkanüle 19 kann für das Einbringen eines Anästhetikums oder einer anderen Arbeitsflüssigkeit in tiefer gelegene Gewebeschichten vorgedrungen werden.
Schließlich zeigt die Fig. 5 ein Komplettierungsteil, das aus einem Stabilisierungshandgriff 21" und einem daran befestigten Absaugrohr 23 besteht. Dieses Absaugrohr 23 ist so bemessen, dass es über die Einspritzkanüle 19 greift und dabei mit der Einspritzkanüle 19 einen inneren Absaugringkanal ausbildet. In diesen Absaugringkanal münden mehrere, am Umfang des Absaugrohres 23 verteilt angeordnete Absaugbohrungen 24. Am distalen Ende besitzt das Absaugrohr 23 eine axiale Bohrung 25, die mit Spiel auf den Durchmesser der Einspritzkanüle 19 in diesem Bereich abgestimmt ist. Das Absaugrohr 23 hat außerdem eine solche Länge, dass die Düsenöffnung 20 der Einspritzkanüle 19 mit einer entsprechenden Länge aus der axialen Bohrung 25 herausragt.

Die neue chirurgische Einrichtung zur Entnahme von Gewebezellen ist universell einsetzbar und ersetzt eine Mehrzahl von speziellen Werkzeugen.
So kann die chirurgische Einrichtung zum Einspritzen eines Anästhetikums eingesetzt werden, wie es normalerweise vor einer Gewebeentnahme üblich ist. Dazu wird zunächst bedarfsweise das erste Komplettierungsteil in Form des Stabilisierungshandgriffs 21 oder das zweite Komplettierungsteil in Form des Stabilisierungshandgriffs 21' mit dem Stabilisierungsrohr 22 auf die Einspritzkanüle 19 geschoben und mit dem Handstück 14 verbunden. Anschließend wird das Wegeventil 12 der Versorgungseinrichtung 1 in die Stellung gebracht, in der die Versorgungspumpe 7 zum Beispiel für das Anästhetikum über die Druckversorgungsleitung 13 mit der Einspritzleitung 15 verbunden ist. Nach dem Eindringen der Einspritzkanüle 19 in das entsprechende Gewebeteil wird die Versorgungspumpe 7 aktiviert und eine vorher abgemessene Menge an dem Anästhetikum eingespritzt.
Die neuen chirurgische Einrichtung kann mit der gleichen Ausführung des Operationshandstückes 2 auch zum Einbringen einer Arbeitsflüssigkeit zum Beispiel zum vorherigen Lösen der Gewebezellen eingesetzt werden. Dazu wird lediglich das Wegeventil 12 in eine Stellung geschalten, in der die Druckversorgungsleitung 13 jetzt mit einem Versorgungsbehälter mit einer entsprechenden gewebelösenden Arbeitsflüssigkeit verbunden wird.

In der Hauptsache wird die neue chirurgische Einrichtung aber zur Entnahme von überflüssigem Fettgewebe oder von vermehrungsfähigen Gewebezellen eingesetzt. Dazu wird das dritte Komplettierungsteil in Form des Stabilisierungshandgriffs 21" mit dem Absaugrohr 23 über die Einspritzkanüle 19 geschoben und mit dem Handstück 14 des Operationshandstückes 1 verbunden. Das Wegeventil 12 wird in die Stellung gebracht, in der die Druckversorgungsleitung 13 mit der Versorgungspumpe 5 für die sterile Trennflüssigkeit 6 verbunden ist.
Nach dem Einbringen des mit der Einspritzkanüle 19 komplettierten Absaugrohres 23 in die entsprechende Gewebeschicht werden die Versorgungspumpe 5 für die Trennflüssigkeit und die Vakuumpumpe 3 in einer aufeinander abgestimmten Förderleistung aktiviert. Dadurch tritt die Trennflüssigkeit in der Art eines von der Achsrichtung abgelenkten Flachstrahles aus der Düsenöffnung 20 der Einspritzkanüle 19 aus und wird in intelligenter Weise in die Zwischenräume der Gewebezellen umgelenkt. Dadurch bleiben die Gewebezellen von der Trennkraft verschont und werden lediglich voneinander abgehoben und getrennt. Die so abgeschälten Gewebezellen werden gleichzeitig und zusammen mit der verbrauchten Trennflüssigkeit durch die Kraft des Vakuums angezogen und durch die Absaugbohrungen 24, den inneren Absaugringkanal und der Absaugleitung 16 des Operationshandstückes 2 in die Unterdruckleitung 9 in den Aufnahmebehälter 4 transportiert. Von dort werden die gesammelten Gewebezellen entsorgt oder nach Bedarf zur Weiterverwendung aussortiert.

### Liste der Bezugszeichen

- 1: Versorgungseinrichtung
- 2: Operationshandstück
- 3: Vakuumpumpe
- 4: Aufnahmebehälter
- 5: Versorgungspumpe
- 6: Versorgungsbehälter für eine sterile Trennflüssigkeit
- 7: Versorgungspumpe
- 8: Versorgungsbehälter für ein Anästhetikum oder eine Arbeitsflüssigkeit
- 9: Unterdruckleitung
- 10: Druckleitung
- 11: Druckleitung
- 12: Schaltbares Wegeventil
- 13: Druckversorgungsleitung
- 14: Handstück
- 15: Einspritzleitung
- 16: Absaugleitung
- 17: Kupplungsstück
- 18: Kupplungsstück
- 19: Einspritzkanüle
- 20: Düsenöffnung
- 21: Stabilisierungshandgriff
- 22: Stabilisierungsrohr
- 23: Absaugrohr
- 24: Absaugbohrung
- 25: Axiale Bohrung

## Patentansprüche

1. Chirurgische Einrichtung zum Einspritzen einer Flüssigkeit und/oder zur Entnahme von Gewebezellen aus einer biologischen Struktur, bestehend
- aus einer Versorgungseinrichtung (1) mit einer Flüssigkeitsstrahleinrichtung zum Einspritzen einer Trennflüssigkeit oder einer Arbeitsflüssigkeit und mit einer Absaugeinrichtung zur Absaugung von abgetrennten oder gelösten Gewebezellen und/oder der Trcnnflüssigkcit bzw. der Arbeitsflüssigkeit und
- aus einem Operationshandstück (2) mit einer inneren Einspritzkantlle (19) und einem äußeren Ahsaugrohr (23), die beide im Bereich des Operationshandstückes (2) einen Absaugringkanal ausbilden, wobei
- das äußere Absaugrohr (23) auf die Einspritzkanüle (19) aufschicbbar und am Operationshandstück (2) feststellbar ausgeführt ist, die Einspritzkanüle (19) eine vordere Düschöffnung (20) besitz und das Absaugrohr (23) mit mehreren, an seinem Umfang verteilt angeordneten Absaugbohrungen (24) ausgestattet ist,
**dadurch gekennzeichnet, dass** das Operationshandstück (2) mit einem Handgriff (14) ausgestattet ist und das Absaugrohr (23) mit einem Handgriff (21") zu einem Komplettierungsteil ausgeführt ist, wobei der Handgriff (14) des Operationshandstückes (2) und der Handgriff (21") des Absaugrohres (23) die gleichen äußeren Abmessungen und eine angepasste Gestallung aufweisen und wobei der Handgriff (14) des Operationshandstückes (2) mit dem Handgriff (21") des Absaugrohres (23) oder wahlweise mit einem weiteren Komplettierungsteil verbindbar ausgeführt ist, das über einen ebenfalls angepassten Handgriff (21, 21') verfügt.

2. Chirurgische Einrichtung nach Anspruch 1, umfassend das weitere Komplettierungsteil in Form eines Stabilisierungshandgriffs (21) zur Stabilisierung der Einspritzkanüle (19).

3. Chirurgische Einrichtung nach Anspruch 1, umfassend
das weitere Komplettierungsteil bestehend aus einem Handgriff (21') und einem offenen Stabilisierungsrohr (22), wobei der Inncndurchmesser des Stabilisicrungsrohres (22) mit Spiel auf den Aaßendurchmesser der Einspritzkanüle (19) abgestimmt ist und die Länge des Stabilisierungsrohres (22) gegenüber der Länge der Einspritzkanüle (19) soweit kürzer ist, dass die Spitze der Einspritzkanüle (19) mit ihrer Düsenöffnung (20) herausragt.

4. Chirurgische Einrichtung nach Anspruch 1,
**dadurch gekennzeichnet, dass** das Absaugrohr (23) eine vordere axiale Bohrung (25) besitzt, die mit Spiel auf den Durchmesser der Einspritzkanüle (19) abgestimmt ist und die Länge des Absaugrohres (23) gegenüber der Länge der Einspritzkanüle (19) soweit kurzer ist, dass die Spitze der Einspritzkanüle (19) mit ihrer Düsenöffnung (20) um einen ausreichenden Betrag herausragt.

5. Chirurgische Einrichtung nach den Ansprüchen 1 bis 4.
**dadurch gekennzeichnet, dass** Flüssigkeitsstrahleinrichtung der Versorgungseinrichtung (1) zur alternativen Versorgung der Einspritzkanüle (19) mit verschiedenen Arbeitsffüssigkciten mit ein oder mehreren Versorgungspumpen (5, 7) und mit einem schaltbaren Wegeventil (12) ausgerüstet ist.

## Claims

1. A surgical device for injecting a fluid and/or for extracting tissue cells from a biological structure, consisting of
- a supply device (1) with a fluid jet means for injecting a separating fluid or a working fluid and with an extracting device for extracting separated or dissolved tissue cells and/or the separating fluid or working fluid and
- an operating handpiece (2) with an inner injection needle (19) and an outer extraction tube (23), the two of which form an annular extraction channel in the area of the operating handpiece (2),
- the outer extraction tube (23) being constructed so as to be capable of being pushed onto the injection needle (19) and secured to the operating handpiece (2), the injection needle (19) having a front nozzle opening (20) and the extraction tube (23) being provided with a plurality of extraction holes (24) distributed around its circumference,
**characterised in that** the operating handpiece (2) is provided with a handle (14) and the extraction tube (23) is constructed with a handle (21") as a completion part, the handle (14) of the operating handpiece (2) and the handle (21") of the extraction tube (23) having the same external dimensions and an adapted design and the handle (14) of the operating handpiece (2) being constructed so as to be connectable to the handle (21") of the extraction tube (23) or optionally to a further completion part, which has a likewise adapted handle (21, 21').

2. A surgical device according to claim 1, comprising the further completion part in the form of a stabilising handle (21) for stabilising the injection needle (19).

3. A surgical device according to claim 1, comprising the further completion part consisting of a handle (21') and an open stabilising tube (22), the internal diameter of the stabilising tube (22) being matched with play to the external diameter of the injection needle (19) and the length of the stabilising tube (22) being shorter than the length of the injection needle (19) to the extent that the tip of the injection needle (19) projects with its nozzle opening (20).

4. A surgical device according to claim 1, **characterised in that** the extraction tube (23) has a front axial bore (25), which is matched with play to the diameter of the injection needle (19) and the length of the extraction tube (23) is shorter than the length of the injection needle (19) to the extent that the tip of the injection needle (19) projects with its nozzle opening (20) by a sufficient amount.

5. A surgical device according to claims 1 to 4,
**characterised in that** the fluid jet device of the supply device (1) is provided, for alternate supply of the injection needle (19) with different working fluids, with one or more supply pumps (5, 7) and with a switchable directional control valve.

## Revendications

1. Installation chirurgicale permettant d'injecter un liquide et /ou de prélever des cellules tissulaires d'une structure biologique, constitué
- d'un dispositif d'alimentation (1) comportant un dispositif à jet de liquide pour injecter d'un liquide de séparation ou un liquide de travail, et d'un dispositif d'aspiration pour aspirer des cellules tissulaires séparées ou dissoutes et /ou du liquide de séparation ou du liquide de travail, et
- d'un élément manuel d'opération (2) comportant une canule d'injection intérieure (19) et un tube d'aspiration extérieur (23) qui forment tous deux, dans la région de l'élément manuel d'opération (2), un canal annulaire d'aspiration,
- le tube d'aspiration extérieur (23) étant exécuté de manière à pouvoir être déplacé sur la canule d'injection (19) et être fixé sur l'élément manuel d'opération (2), la canule l'injection (19) possédant une ouverture en forme de buse avant (20) et le tube d'aspiration (23) étant équipé de plusieurs trous d'aspiration répartis sur sa périphérie (24),
**caractérisée en ce que**
l'élément manuel d'opération (2) est muni d'une poignée (14) et le tube d'aspiration (23) est exécuté avec une poignée (21") en une partie complémentaire, la poignée (14) de l'élément manuel d'opération (2) et la poignée (21") du tube d'aspiration (23) ont les mêmes dimensions extérieures en présentant une configuration adaptée, et la poignée (14) de l'élément manuel d'opération (2) est exécutée de manière à pouvoir être reliée avec la poignée (21") du tube d'aspiration (23) ou, au choix, avec une autre partie complémentaire qui comporte une poignée (21, 21') également adaptée.

2. Installation chirurgicale selon la revendication 1, comportant l'autre élément complémentaire sous forme d'une poignée de stabilisation (21) pour stabiliser la canule d'injection (19).

3. Installation chirurgicale selon la revendication 1, comprenant l'autre partie complémentaire constituée d'une poignée (21') et d'un tube de stabilisation ouvert (22), le diamètre intérieur du tube de stabilisation (22) étant ajusté avec du jeu sur le diamètre extérieur de la canule d'injection (19), et la longueur du tube de stabilisation (22) étant suffisamment plus courte que la longueur de la canule d'injection (19), pour que la pointe de la canule d'injection (19) dépasse avec son ouverture en forme de buse (20).

4. Installation chirurgicale selon la revendication 1,
**caractérisée en ce que**
le tube d'aspiration (23) possède un perçage axial avant (25) ajusté avec du jeu sur le diamètre de la canule d'injection (19) et la longueur du tube d'aspiration (23) est suffisamment plus courte que la longueur de la canule d'injection (19) pour que la pointe de la canule d'injection (19) dépasse d'une longueur suffisante avec son ouverture en forme de buse (20).

5. Installation chirurgicale selon les revendications 1 à 4,
**caractérisée en ce que**
le dispositif à jet de liquide du dispositif d'alimentation (1) est équipé pour l'alimentation alternative de la canule d'injection (19) avec différents liquides de travail, d'une ou de plusieurs pompe(s) d'alimentation (5,7) et d'une vanne à plusieurs voies (12) commutable.
